# EUROPEAN PATENT APPLICATION

(11) **EP 2 157 176 A1**
(43) Date of publication of application: **24.02.2010**
(21) Application number: 08162703.6
(22) Date of filing: 20.08.2008
(51) Int. Cl.: C12N 9/64, C12N 15/57, A61K 38/48

(54) **Protein C Variants**

(71) Applicant: The Provost, Fellows and Scholars of the College of the Holy and Undivided Trinity of Queen Elizabeth near Dublin, Dublin 2 (IE)
(72) Inventor: O'Donnell, James Stewart, Dublin 6 (IE); Preston, Roger J. S., Dublin 6 (IE); Johnson, Jennifer, County Dublin (IE)
(74) Representative: Hally, Anna-Louise

(57) **Abstract**

The present invention relates to variant of protein C and the corresponding activated protein C and uses thereof.

## Description

### INTRODUCTION

The present invention relates to variant of protein C and the corresponding activated protein C and uses thereof.

Protein C is serine protease and a member of the class of vitamin K-dependant serine protease coagulation cofactors. Protein C is inactive and requires proteolytic activation to regulate blood coagulation, in the form of activated protein C, through a natural feedback mechanism.

Protein C is made in vivo in the liver as a single polypeptide of 461 amino acids., comprising a serine protease domain, two epidermal growth factor (EGF) domains, and a Gla domain. Situated at the protein C N terminus, the Gla domain consists of 45 amino acid residues. Of these, 9 glutamic acid (Glu) residues are post-translationally modified to gamma-carboxyglutamic acid (Gla) residues (Preston et al (2006) "Multifunctional Specificity of the Protein C/Activated protein C Gla Domain" J Biological Chemistry 281 (39): 28850-28857).

It is well known that the precursor molecule protein C undergoes multiple post-translational modifications to produce circulating 2-chain zymogen that is activated in vivo by thrombin to produce activated protein C. Thrombin binds to thrombomodulin, a membrane-bound thrombin receptor on the luminal surface of endothelial cells, thereby blocking the procoagulant activity of thrombin and enhancing its anticoagulant properties, i.e. activating protein C.

Activation of protein C results in the release of a small 12-residue peptide near the active site of the molecule. There are no other known structural alterations. Activation of protein C is required to enable both its anticoagulant and cytoprotective functions.

Activated protein C (APC) is the active plasma anticoagulant protein which circulates in the plasma in its inactive form, protein C. When protein C is activated by the thrombin/thrombomodulin complex as described above, APC cleaves and inactivates the procoagulant factors Va and VIIIa which are involved in the coagulation cascade.

As well as this anticoagulant function it has recently been shown that APC has important anti-inflammatory and cytoprotective functions. This was highlighted by the use of APC in the Protein C Evaluation in Severe Sepsis (PROWESS) trial (Bernard et al (2001) "Efficacy and safety of recombinant human activated protein C for severe sepsis" N Engl J Med Vol. 344, No. 10: 699-709) where APC was found to significantly improve the survival of patients with severe sepsis. The therapeutic potential of APC was highlighted in the Protein C Evaluation in Severe Sepsis (PROWESS) study. Intravenous infusion of recombinant APC (24µg/kg per hour for 96 hours) significantly reduced overall mortality (relative risk reduction, 19.4%) in adult patients with severe sepsis. In contrast, treatment with other anticoagulants, notably antithrombin and tissue factor pathway inhibitor, have no such effect on survival. APC mediates the anti-inflammatory effects through the inhibition of cytokine generation (e.g. TNF and IL-1).

Thus, recombinant APC is used in treatment of patients with severe sepsis. However, although recombinant APC is widely used to treat severe sepsis, large cohort studies have demonstrated that the therapeutic benefits of APC are offset by a significant associated bleeding risk. For example in the Protein C Evaluation in Severe Sepsis (PROWESS) study, serious bleeding complications were observed in 3.5% of all patients treated with APC. This bleeding risk limits the dose and the duration of APC that can be safely administered in clinical practice. Thus, the risk of bleeding due to the anticoagulant function of APC is one of the major downsides to administration of APC and limits the dose and duration of APC that can be safely utilized in clinical practice. Thus, to date the use of APC in clinical settings has been limited due to this significant and potentially life threatening treating side effect.

Recent studies have shown that the therapeutic benefit of APC in these patients is mediated through its anti-inflammatory and cytoprotective properties and not due to its anticoagulant activity. Accumulating evidence from different animal disease models suggests that the cytoprotective signaling activity of APC may be of greater significance than its anticoagulant function in protection against various disease states, including severe sepsis, inflammatory bowel disease and ischemic stroke. For example, APC demonstrated significant neuroprotective effects in rat and murine stroke models, and was also recently shown to ameliorate experimental autoimmune encephalomyelitis in mice. Furthermore, APC was protective in a murine model of diabetic nephropathy by inhibition of endothelial and podocyte apoptosis, whereas anticoagulation with low molecular weight heparin had no such beneficial effect. Each of these animal studies (as listed below) demonstrated a consistent and critical role for the cytoprotective and anti-inflammatory properties of APC.
Scaldaferri et al (2007) J. Clin. Invest. "Crucial Role of the protein C pathway in governing microvascular inflammation in inflammatory bowel disease", 117(7):1951-1960;
Cheng et al (2003) Nat Med "Activated protein C blocks p53-mediated apoptosis in ischemic human brain epithelium and is neuroprotective". 9(3):338 - 342;
Isermann et al (2007) Nat Med "Activated protein C protects against diabetic nephropathy by inhibiting endothelial and podocyte apoptosis"13(11):1349-1358;
Han et al (2008) Nature "Proteomic analysis of active multiple sclerosis lesions reveals therapeutic targets", 451:1076-1081; and
Shibata et al (2001) Circulation "Anti-inflammatory, antithrombotic and neuroprotective effects of activated protein in a murine modle of focal ischemic stroke" 103:1799-1805.

Consequently, research has sought to develop APC variants that retain the beneficial cytoprotective and anti-inflammatory effects, but have a reduced anticoagulant function.

Mosnier *et al*. demonstrated that clustered alanine mutations (RR229/230AA) and (KKK191-193AAA) in two surface loops of the APC serine protease domain disrupted the anion binding site on APC for FVa exosite binding (Mosnier, L. O., Yang, X. V., and Griffin, J. H. (2007) J Biol Chem 282(45), 33022-33033*)*.

Using an alternative approach, Bae *et al* engineered a disulfide bond (Cys67-Cys82; chymotrypsin numbering) within the APC serine protease domain, which prevented Ca2+ binding to the functionally critical Ca2+ binding 70-80 loop (Bae, J. S., Yang, L., Manithody, C., and Rezaie, A. R. (2007) J Biol Chem 282(12), 9251-9259*)*.

Therefore, protein C variants and corresponding activated protein C variants with reduced anti-coagulant activity but retaining normal cytoprotective and anti-inflammatory activity would be of great use in the treatment of severe sepsis and other diseases.

The present invention is directed to providing an alternative protein C and corresponding activated protein C variant.

### STATEMENT OF THE INVENTION

According to a first aspect of the invention, there is provided a protein C variant or activated protein C variant which has reduced or eliminated lipid co-factor enhancement of activated protein C (APC) anticoagulant function.

According to a second aspect of the invention, there is provided a protein C variant of the invention for use in therapy.

According to a third aspect of the invention, there is provided a method of treating an anti-inflammatory condition comprising the steps of administering the protein C variant of the invention to a patient in need thereof.

### DETAILED DESCRIPTION

The present invention is directed to a protein C variant, and corresponding activated protein C (APC) variant, which has a reduced anticoagulant activity, due to reduced sensitivity to specific lipids/lipid co-factors which normally enhance APC anticoagulant activity in-vivo, whilst maintaining the therapeutic cytoprotective and/or anti-inflammatory properties. Thus, the present invention provides a means for utilising the important cytoprotective and/or anti-inflammatory properties of protein C/APC whilst minimizing/reducing the undesirable anti-coagulation properties.

Advantageously, we have found that at a concentration of approximately 20nM, the present invention can provide an approximately 6-fold (P<0.0005) reduction in anticoagulant activity compared to wild-type APC whilst maintaining and even enhancing the cytoprotective and/or anti-inflammatory properties.

It will be understood that the protein C variant of the invention reduces the anticoagulant activity of APC through an entirely different mechanism than known protein C/APC variants by disrupting interaction with specific lipid co-factors. Protein C variants known to date were created by a targeted mutation of a different part of APC structure (e.g. serine protease domain) and thus, APC anticoagulant activity was affected by a different mechanism.

This is the first mutation in protein C/APC to be identified which can specifically interfere with the ability of APC to interact with lipid co-factors. Elimination of lipid cofactor enhancement of APC anticoagulant function represents a novel and effective strategy to dissociate the anticoagulant and cytoprotective functions of protein C/APC.

Lipid cofactor enhancement of APC function generally occurs through the interaction of protein S and APC which is enhanced by phospholipids, including phosphatidylethanolamine (PE) and glucosylceramide (GlyCer). We have modulated this interaction to result in a protein C variant and APC variant which have reduced anti-coagulant effects whilst maintaining and even enhancing the therapeutic cytoprotective and/or anti-inflammatory properties.

Although, the mechanism by which protein S exerts its cofactor activity on APC in the presence of negatively-charged phospholipids is not fully elucidated, essentially protein S enhances APC affinity for phospholipids and is required for optimal alignment of the APC active site for substrate cleavage. Protein S has also been reported to remove factor Xa (FXa) protection of FVa in the prothrombinase complex.

Other lipid cofactors of APC include but are not limited to Cardiolipin and/or High Density lipoprotein (HDL). Thus, these additional lipid co-factors may also be targeted by the present invention.

Advantageously, the protein C variant of the invention and corresponding APC variant, will result in a reduced bleeding risk when administered in-vivo. This reduced bleeding risk will allow for the protein C variant and corresponding APC variant to be used in a clinical setting at higher doses, and for longer durations of administration compared to normal wild-type protein C/APC.

A further embodiment of the invention is directed to a pharmaceutical composition comprising protein C variant or activated protein C variant according to the invention together with a suitable pharmaceutical excipient.

Another aspect of the invention provides an activated protein C variant or pharmaceutical composition according to the invention in a form suitable for intravenous administration or any other suitable form of administration.

A still further aspect of the invention provides a protein C variant or activated protein C variant of the invention use in therapy.

A yet further aspect of the invention is directed to the use of protein C variant or activated protein C variant of the invention for the manufacture of a medicament for the treatment of anti-inflammatory conditions, such as severe sepsis, inflammatory bowel disease or ischemic stroke.

A still further aspect of the invention is directed to a method of treating an anti-inflammatory condition comprising the steps of administering the protein C variant or activated protein C variant to a patient in need thereof.

The protein C or activated protein C variant of the invention is ideally used in the treatment of various anti-inflammatory conditions. The following lists diseases and disorders which may be treated by the invention including but not limited to:
Diabetic nephropathy;
Severe Sepsis;
Inflammatory bowel disease, including Crohn disease and ulcerative colitis (UC);
Multiple sclerosis (MS);
Ischemic Stroke;
intravascular coagulation, including thrombotic stroke;
deep vein thrombosis;
pulmonary embolism;
peripheral arterial thrombosis;
emboli originating from the heart or peripheral arteries;
acute myocardial infarction;
disseminated intravascular coagulation;
acute pre or postcapillary occlusions, including transplantations or retina thrombosis;
vasculitis;
renal ischemia;
pancreatitis;
rheumatoid arthritis;
ischemic renal failure;
insulin-dependent diabetes mellitus;
pancreatic;
psoriasis;
Hashimoto's thyroiditis;
Graves disease;
systemic lupus;
erythematosus;
autoimmune gastritis;
fibrosing lung disease;
HIV-induced lymphoma;
fulminant viral hepatitis B;
fulminant viral hepatitis C;
chronic hepatitis;
chronic cirrhosis;
H. pylori-associated ulceration;
Atherosclerosis;
cytoprotection during cancer treatment;
chronic glomeruonephritis;
osteoporosis;
aplastic anemia;
myelodysplasia;
Alzheimer's disease; and/or
Parkinson's disease.

According to a preferred embodiment of the invention, there is provided a mutant APC protein (e.g. APC-I18V), in which only a single amino acid residue of APC is altered which has a significantly reduced anticoagulant activity whilst maintaining the therapeutically-desirable cytoprotective and anti-inflammatory properties.

In the protein C form and in the activated form, APC-I18V is a substitution in the Gla domain at position 18 from isoleucine to valine.

APC-I18V differs from wildtype APC only in respect to a single amino acid residue substitution in the γ-carboxyglutamic acid (Gla) domain of APC, I18V. This APC Gla domain plays a critical role in modulating the interaction of APC with a number of specific lipid co-factors, notably phosphatidylethanolamine (PE) and glucosylceramide (GlyCer) respectively, on the endothelial cell surface. The interaction of the APC Gla domain with PE and GlyCer is of major functional importance, in that it enhances the anticoagulant activity of APC by enabling APC to associate with Protein S and continue with the protein C anticoagulant pathway.

The protein C/APC Gla domain is situated at the N-terminal end of the protein C/APC light chain. It is composed of 45 amino acid residues, of which 9 glutamate residues are post-translationally modified to gamma-carboxyglutamate prior to release into plasma. These Gla residues co-ordinate Ca²⁺ binding, which enables the Gla domain to adopt a conformation that is functionally active. Once in this conformation, the Gla domain mediates APC binding to phospholipids, the endothelial protein C receptor (EPCR), and protein S.

The protein C DNA sequence is well known and has been published in Foster et al. (1985) PNAS 82:4763-4677. The protein C amino acid sequence was published in Beckmann RJ et al. (1985) Nucleic Acids Res 13:5233-5247. Protein C/APC Gla domain crystal structure has been published in Oganesyan et al (2002) J Biol Chem. Jul 12;277(28):24851-4 and the Gla domain less APC crystal structure has been published in Mather et al (1996) EMBO J. Dec 16;15(24):6822-31.

We have demonstrated that the APC-I18V substitution in the Gla domain of APC significantly decreases APC sensitivity for both PE and GlyCer, and thereby interferes with APC anticoagulant activity, by reducing or eliminating interaction with protein S. However, this mutation does not influence the anti-inflammatory or cytoprotective properties of APC.

Advantageously, we have found that at a concentration of approximately 20nM APC-I18V is approximately 6-fold (P<0.0005) less active as an anticoagulant compared to wild-type APC but maintains and even enhancing the cytoprotective and/or anti-inflammatory properties.

Consequently, the present invention is directed to the first APC variant which specifically disrupts the critical interaction between APC and lipids. This APC variant has significantly reduced anticoagulant properties (and therefore bleeding risk), yet entirely retains its cytoprotective and anti-inflammatory properties. Consequently, the variant of the present invention APC-I18V in particular constitutes a novel and exciting potential therapeutic advance.

The present invention will now be described with reference to the following nonlimiting figures and examples.

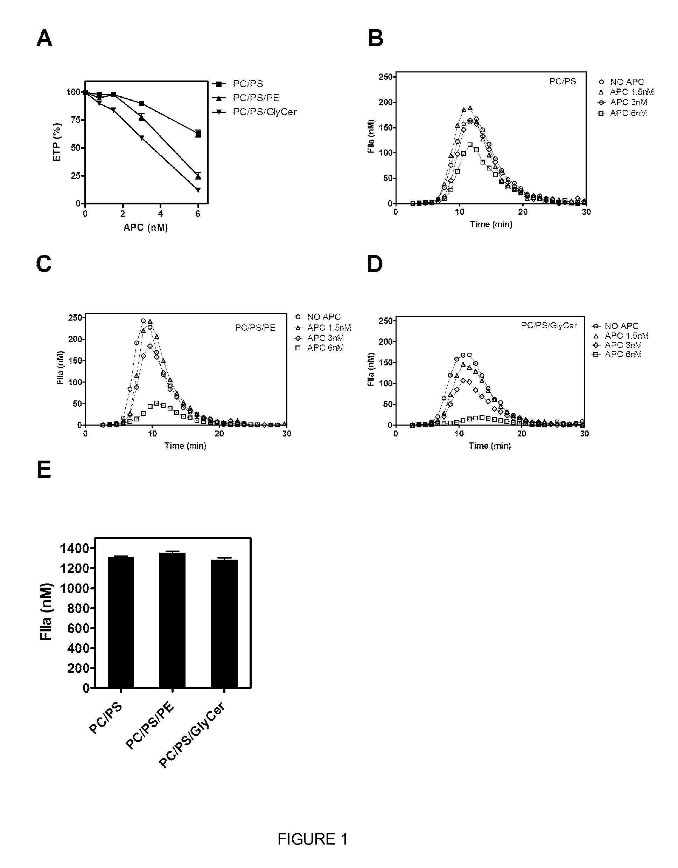

Figure 1 shows the effect of PE and GlyCer in phospholipid vesicles on thrombin generation in the presence of wild-type APC. (A) Thrombin generation in the presence of 10µM phospholipid vesicles (PC/PS, ■) containing either PE, (PC/PS/PE, ▲) or GlyCer (PC/PS/GlyCer, ▼), 5pM sTF and protein C-deficient plasma with wild-type APC (1.25-6nM APC), as described in 'Experimental Procedures'. Thrombograms were subsequently generated for thrombin generation in the presence of PC/PS (B), PC/PC/PE (C), and PC/PS/GlyCer (D) in the presence of the indicated wild-type APC concentration. (E) Thrombin generation was measured under the same experimental conditions with each phospholipid vesicle composition. The values represent mean ETP±SEM from at least 3 determinations.

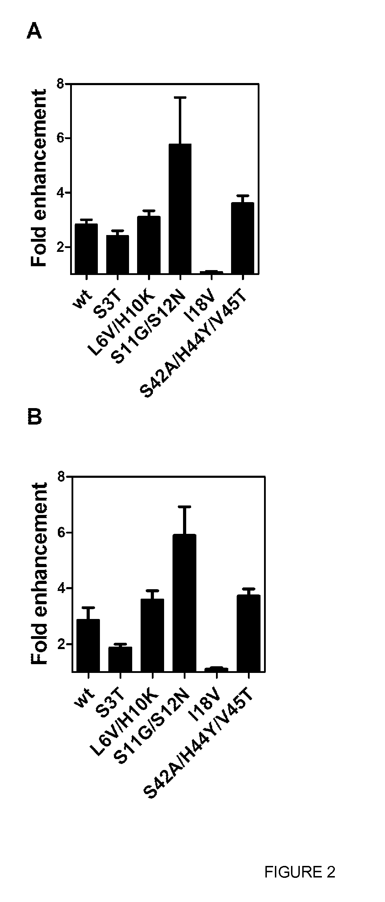

Figure 2 shows that the APC variant APC-I18V has reduced sensitivity to PE and GlyCer compared to wild-type-APC. Thrombin generation was determined in protein C-deficient plasma in the presence of 10µM phospholipid vesicles (PC/PS, PC/PS/PE or PC/PS/GlyCer) 5pM soluble tissue factor and wild-type APC and variant APC at 10nM (except APC-S11G/S12N at 5nM). The fold-enhancement of APC anticoagulant activity observed with (A) PC/PS/PE or (B) PC/PS/GlyCer compared to PC/PS was determined. The values represent the mean ETP±SEM (%) from 3 determinations. Unpaired two-tailed t-tests were used to determine significance of APC-I18V compared to wild-type APC (* P<0.01, ** P<0.009).

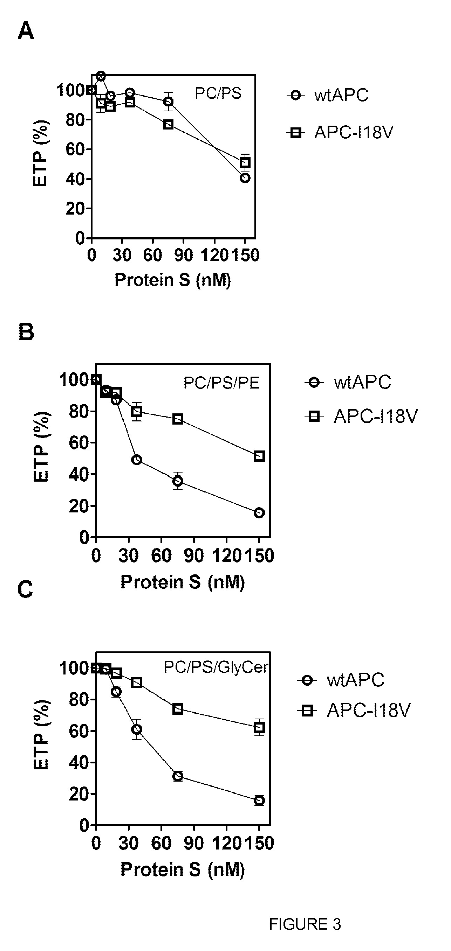

Figure 3 shows that PE and GlyCer enhance wild-type APC sensitivity to its cofactor protein S cofactor, while APC-I18V is insensitivity to this enhancement. The ability of wild-type APC (○) and APC-I18V (□) to inhibit sTF (5pM) induced thrombin generation in protein S-deficient plasma was assessed in the presence of plasma-purified protein S (7.5-150nM) with 10µM phospholipid vesicles PC/PS (A), PC/PS/PE (B), AND PC/PS/GlyCer (C). Values represent mean ETP ± SEM (%) of 3 determinations.

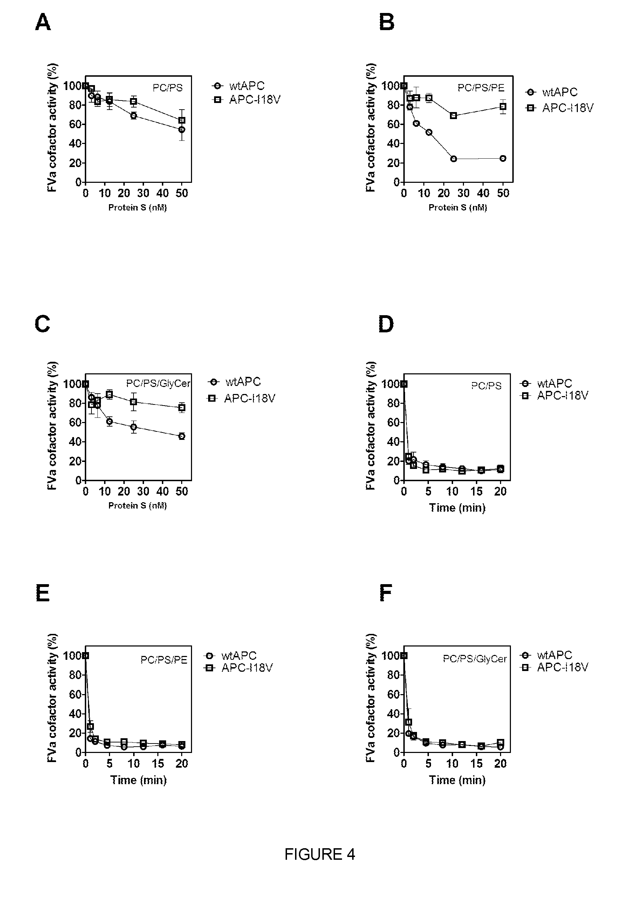

Figure 4 shows that PE and GlyCer increase APC sensitivity to protein S activity during FVa proteolysis. (A-C) FVa inactivation in presence of protein S: Human protein purified protein S (3.25-50nM) was incubated with wild-type APC (○) or APC-118V (□), FVa 7nM and 25µM phospholipid vesicle PC/PS (A), PC/PS/PE (B), or PC/PS/GlyCer (C). After 2 minute incubation, an aliquot was removed and added to a prothrombinase assay (1nM FXa, 0.5µM prothrombin and 25µM phospholipids vesicles final concentrations at 37°C for 3 minutes) to assess FVa cofactor activity. (D-F) Time-dependent FVa inactivation by APC in absence of protein S: FVa (1nM) was incubated with wild-type APC (○) or APC-I18V (□) (both 2nM) in the presence of 25µM phospholipid vesicles containing PC/PS (A), PC/PS/PE (B), or PC/PS/GlyCer (C). The FVa cofactor activity at specified time points was determined using a prothrombinase assay (1nM FXa, 0.5µM prothrombin and 25µM phospholipids vesicles final concentrations at 37°C for 3 minutes). Values represent mean residual FVa cofactor activity ± SEM (%) for 3 determinations.

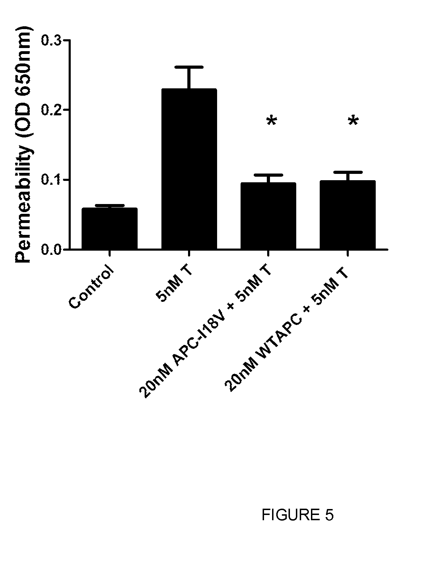

Figure 5 shows that APC-18V exhibits normal endothelial barrier protection. The effect of wild-type APC and APC-I18V on the endothelial cell barrier was determined. EaHy926 cells were pre-incubated with 20nM of each APC for 3 hours. Untreated cells (Control) were used as a negative control. EaHy926 cells were then treated with 5nM thrombin in serum-free media for 10mins and endothelial barrier permeability assed after 30minutes using Evans Blue-BSA. Unpaired 2-tailed t-tests were used to determine significance (* p<0.01 compared to thrombin-only treated Eahy926 cells).

### Example

### METHODS

### Generation of Recombinant Protein C Variants

Recombinant protein C variants were generated using standard site-directed mutagenesis techniques. Expression vectors for each protein C variant were used to transfect human embryonic kidney 293 cells (European Collection of Cell Cultures, Wiltshire, UK) and transfected cells isolated by G418 selection (Invitrogen). Serum-free conditioned medium containing each recombinant protein C was buffer-exchanged against 20mM Tris-HCl (pH 7.4), 150mM NaCl. Protein C was purified from cell culture medium by ion-exchange chromatography using a Mono-Q 5/50 column (Amersham Biosciences)with a gradient elution using 20mM Tris-HCl, pH 7.4, 150mM NaCl, 30mM CaCl₂ as previously described. Recombinant protein C antigen concentration was determined using a protein C ELISA kit (Hyphen-BioMed). The purity of all recombinant proteins was confirmed by SDS-PAGE and Coomassie staining, and Western blotting was performed using a horseradish peroxidase-conjugated polyclonal anti-protein C antibody (Dako, Ely, UK). Wild-type APC and APC variants were created using PROTAC activation as previously described (Preston et al (2006) "Multifunctional Specificity of the Protein C/Activated protein C Gla Domain" J Biological Chemistry 281 (39): 28850-28857) and active-site titrated using APC of known concentration.

### Phospholipid Vesicle Preparation

Lyophilised pre-formulated phospholipids (DOPC/DOPS, 80%:20% w/w, DOPC/DOPS/DOPE 60%:20%:20% w/w and DOPC/DOPS/GlyCer 60%:20%:20% w/w, Avanti Polar Lipids AL, USA) were hydrated using distilled water to 10mg/ml and extruded 11 times through a 0.1µm polycarbonate membrane (Avanti Polar Lipids) to achieve unilamellar vesicles. Phospholipids vesicles were then stored at 4°C and discarded after 5 days storage.

### Determination of APC Anticoagulant Activity in Protein C-Deficient Plasma

The effect of phospholipid vesicles of different composition upon thrombin generation in the presence of wild-type and variant APC anticoagulant activity was assessed using a Fluoroskan Ascent Plate Reader (Thermo Lab System, Helsinki, Finland) in combination with Thrombinoscope software (Thrombinoscope). 80µl protein C-deficient plasma (Hyphen-Biomed) was incubated with the indicated phospholipid vesicles (either PC/PS, PC/PS/PE or PC/PS/GlyCer, all 10µM, final concentration) and 5pM soluble tissue factor (sTF, Dade-Innovin) in the presence or absence of wild type or variant APC (1.25-6nM). Thrombin generation was initiated using fluorogenic thrombin substrate (Z-Gly-Gly-Arg-AMC.HCL) along with 100mM CaCl₂ (final concentration) into each well. A thrombin calibration standard (Thrombinoscope) was used to quantify thrombin generation. Measurements were recorded every 20 seconds and the endogenous thrombin potential (ETP) of each reaction was calculated using Thrombinoscope software. Experiments were performed in triplicate, and data reported as mean ETP +/- SEM.

### Protein S Enhancement of APC Anticoagulant Activity in Protein S-Deficient Plasma

The effect of PE and GlyCer upon protein S cofactor enhancement of APC anticoagulant activity was assessed using a similar assay to the one described above. Briefly, 80µl protein S-deficient plasma (Hyphen-Biomed) was incubated with wild-type or variant APC (10nM) in the presence or absence of plasma-purified protein S (7.5-150nM, Hematologic Technologies Inc.) Thrombin generation was initiated as described above, and the ETP determined. All experiments were performed in triplicate and data plotted as mean ETP +/- SEM.

### Determination of Protein S-Enhanced Proteolysis of FVa by APC in the Presence of Different Phospholipids

Protein S-enhanced proteolysis of FVa by APC was determined as previously described. Human protein S (3.25-50nM) was incubated with 8nM wild type or variant APC, 28nM FVa and 80µM phospholipids vesicles in 40mM Tris-HCl (pH 7.4), 150mM NaCl, 3mM CaCl₂, 0.3% (w/v) bovine serum albumin (2nM APC, 7nM FVa, and 20µM phospholipids vesicles, final concentrations) for 2 minutes at 37°C. Following this incubation, a 2µl aliquot was added to 1nM FXa, 0.5µM prothrombin and 25µM phospholipids vesicles (final concentrations) at 37°C for 3 minutes, and the reaction stopped with 5µl of ice cold 0.5M EDTA. 40µl of the reaction mixture was then removed and incubated with 40µl of 2mM thrombin chromogenic substrate BIOPHEN CS-01(38) (Hyphen-Biomed) to assess thrombin generation. Experiments were performed in triplicate and data plotted as mean residual FVa cofactor activity +/- SEM.

### Determination of Protein S Independent APC-Mediated Factor Va Proteolysis

FVa degradation by APC was assessed as previously described. 8nM APC was incubated at 37°C with 80µM phospholipids vesicles and 4nM FVa (Hematologic Technologies Inc.) in 40mM Tris-HCl, 140mM NaCl, 3mM CaCl₂ and 0.3% w/v bovine serum albumin (2nM APC, 20µM phospholipids and 1nM FVa, final concentrations). At specified time points over 20 minutes, 2µl aliquots were removed and added to a prothrombinase mixture (25µM phospholipids, 1nM factor Xa and 0.5µM prothrombin, final concentrations, Hematologic Technologies Inc.) for 3 minutes. Each reaction was then stopped using 5µl of ice-cold 0.5M EDTA. 40µl of the reaction mixture was removed and incubated with 40µl of 2mM thrombin chromogenic substrate Biophen CS-01 (38) (Hyphen-Biomed) to assess thrombin generation. The rate of chromogenic substrate cleavage was measured at 405nm using a plate reader. All experiments were performed in triplicate, and data represented as the mean residual FVa cofactor activity +/- SEM.

### Measurement of Endothelial Cell Barrier Protection by APC

Endothelial cell barrier permeability was determined as described previously. Briefly, EAhy926 cells (gift of Dr. C. Edgell, University of North Carolina, Chapel Hill, NC) were grown to confluence on polycarbonate membrane transwells (Costar, 3µM pore size, 12mm diameter) and incubated with 20nM wild-type or variant APC After 3 hours, the cells were treated with thrombin (Hematologic Technologies Inc.) in serum-free media for 10 minutes. The cells were washed and incubated with 0.67mg/ml Evans Blue with 4% bovine serum albumin (BSA, Sigma). Changes in endothelial cell barrier permeability were determined by following the increase in absorbance at 650nm in the outer chamber over time due to the transmigration of Evans Blue-BSA. Experiments were performed in triplicate and plotted as the mean +/- SEM.

### RESULTS

### PE and GlyCer enhance APC anticoagulant activity, but do not affect thrombin generation

To assess the contribution of PE and GlyCer lipids upon APC anticoagulant activity, thrombin generation in the presence of phospholipid vesicles containing PC/PS, PC/PS/PE and PC/PS/GlyCer (80%:20%, 60%:20%:20% and 60%:20%:20% respectively) and APC was assessed. In the presence of PC/PS phospholipid vesicles alone, APC anticoagulant activity was slow, reducing thrombin generation (as determined by ETP) by only 40% at the highest APC concentration tested (6nM) (Fig 1A and 1B). However, APC anticoagulant activity was markedly enhanced by the addition of either PE or GlyCer to phospholipid vesicles containing PC/PS. APC impairment of thrombin generation was enhanced ∼3-fold in the presence of phospholipid vesicles containing PC/PS/PE compared with vesicles containing PC/PS alone (P<0.0001, Fig. 1A, B and C). Furthermore, APC anticoagulant activity in the presence of PC/PS/GlyCer was 4.3-fold more active than when phospholipid vesicles containing only PC/PS were used (P<0.0001, Fig 1A and D). In the absence of APC, however, there was no significant effect upon thrombin generation when tested in the presence of any of the phospholipid vesicle mixtures (Fig. 1E). These results suggest that both PE and GlyCer specifically enhance APC anticoagulant activity, yet have minimal effect upon thrombin generation in plasma.

### Identification of key APC Gla domain residue(s) that mediate PE and GlyCer enhanced APC anticoagulant activity

Prothrombin functional activity is not enhanced by the presence of PE or GlyCer. Consequently, Gla domain amino acids present in the APC Gla domain, but not shared with the prothrombin Gla domain, may be predicted to mediate APC specific functional enhancement by PE/GlyCer. To determine the APC residues responsible for PE and GlyCer specific-enhanced APC anticoagulant activity, a series of APC Gla domain variants were used in which APC Gla domain residues not shared with the prothrombin Gla domain were substituted with their prothrombin amino acid equivalent. The majority of APC variants tested (APC-S3T, APC-L8V/H10K and APC-S42A/H44Y/V45T) were found to be enhanced by PE (Fig. 2A) or GlyCer (Fig. 2B) in a similar manner to that of wild-type APC. APC-S11G/S12N exhibited markedly higher anticoagulant activity independent of the phospholipid vesicle composition used (Figure 2). Interestingly, APC-I18V anticoagulant activity was similar to that of wild-type APC with PC/PS vesicles, but was not enhanced by the presence of both PE and GlyCer in PC/PS vesicles. This indicates Ile-18 in the APC Gla domain plays a crucial role in mediating both PE- and GlyCer specific enhancement of APC anticoagulant activity.

### Ile-18 in the APC Gla domain is required for PE- and GlyCer-mediated protein S cofactor enhancement

Phospholipid vesicles containing PE and/or GlyCer have both been previously described to enhance protein S cofactor function with APC. Therefore, we assessed the ability of wild-type APC and APC-I18V to be enhanced by protein S in the presence of phospholipid vesicles containing either PC/PS, PC/PS/PE or PC/PS/GlyCer using a protein S-sensitive thrombin generation assay. In the presence of PC/PS and APC, increasing protein S concentration in protein S-deficient plasma resulted in a slow decrease in thrombin generation, irrespective of whether wild-type or APC-I18V was used (Figure 3A). However, when the same experiment was performed in the presence of PC/PS/PE or PC/PS/GlyCer, thrombin generation was impaired by wild-type APC at 3-4-fold lower protein S concentration than that observed when PC/PS vesicles alone were used (IC₆₀ for PC/PS=127nM, PC/PS/PE=31.5nM, PC/PS/GlyCer=37.5nM, Figure 3B and 3C). This suggests that the molecular mechanism through which both PE and GlyCer increases APC anticoagulant activity is via increased sensitivity to its cofactor protein S. The APC-I18V variant, whose anticoagulant activity is not stimulated by the presence of PE and GlyCer, did not exhibit a similarly increased sensitivity to protein S in the presence of PE or GlyCer (Fig. 3B and 3C; IC₆₀ for PC/PS=133nM, PC/PS/PE=114nM, PC/PS/GlyCer=150nM), suggesting that for protein S to exhibit full APC cofactor activity, specific lipid cofactors must be present on the cell surface to interact with APC.

### PE and GlyCer do not enhance FVa proteolysis by APC-118V in the presence of protein S

To investigate the role of PE/GlyCer upon FVa proteolysis by APC, the rate of FVa inactivation in the presence of each of the phospholipid vesicle compositions was examined. Protein S-mediated FVa proteolysis by both wild type APC and APC-118V was slow in the presence of PC/PS phospholipid vesicles only, retaining ∼60% residual FVa cofactor activity at the highest protein S concentration tested (Figure 4A). However, FVa proteolysis by wild-type APC was significantly increased by the presence of both PE (3.5-fold) and GlyCer (2-fold) (Fig. 4B and 4C; IC₅₀ for PC/PS=50nM protein S, PC/PS/PE=13.4nM protein S, PC/PS/GlyCer=34.5nM protein S). However, APC-I18V, in keeping with the protein S-dependent plasma assay, did not display this increased rate of FVa proteolysis, and was not enhanced by the presence of PE and GlyCer (Fig. 4-C), suggesting that PE and GlyCer are important for protein S cofactor function during APC-mediated FVa proteolysis, and this enhancement is lost when wild-type APC is replaced with the APC-I18V variant. Interestingly, PE and GlyCer did not to enhance FVa proteolysis by APC when protein S was absent (Fig. 4D-F). Furthermore, the rate of FVa proteolysis by APC-I18V identical to that of wild-type APC, indicating that APC anticoagulant activity in the absence of protein S is not affected by the presence of PE or GlyCer.

### APC-I18V retains normal endothelial barrier protection function despite loss of PE/GlyCer enhancement

To investigate whether PE and/or GlyCer influence the APC signaling properties, a thrombin-induced endothelial cell barrier permeability assay was used to determine whether APC-I18V, which is unaffected by PE or GlyCer in anticoagulant assays, retains the ability to mediate PAR-1 signaling in a similar fashion to wild-type APC. Thrombin incubation was found to induce a significant increase in barrier permeability (Fig. 5), as previously described. However, when cells were pre-treated with wild-type APC, there was a significant enhancement in barrier integrity and attenuation of the thrombin-induced phenotype (P<0.05, wild-type APC treated versus thrombin only treated, Fig. 5). There was an identical protection phenotype observed when cells were pretreated with APC-I18V (P<0.05, APC-I18V treated versus thrombin only treated). Collectively, these results suggest APC interaction with either PE or GlyCer containing phospholipid vesicles, whilst critical for APC anticoagulant activity, does not affect APC signaling through PAR-1 on endothelial cells.

### CONCLUSION

APC-I18V was identified following screening of a series of different candidate APC Gla domain mutants for their sensitivity to PE and GlyCer.

We observed that when PE and GlyCer are present wild-type APC has increased sensitivity to protein S. Compared to wild-type APC, APC-I18V exhibited markedly reduced sensitivity to PE and GlyCer.

However, APC-I18V does not exhibit this increased sensitivity to protein S in the presence of either PE or GlyCer respectively. Although APC-I18V had reduced sensitivity to lipid co-factors, this did not affect its EPCR-PAR-1 signalling capabilities.

APC-I18V was found to exhibit similar cytoprotective properties compared to wild-type APC.

## Claims

1. Protein C variant which in activated form has reduced or eliminated lipid co-factor enhancement of activated protein C (APC) anticoagulant function.

2. Protein C variant according to claim 1 with the same or enhanced cytoprotective and/or anti-inflammatory properties relative to wild type protein C.

3. Protein C variant according to claim 1 or claim 2 wherein the APC anticoagulant activity is reduced or eliminated by disrupting the interaction of APC with lipid co-factors, preferably present on the endothelial cell surface.

4. Protein C variant according to any of the preceding claims which has reduced sensitivity to lipid co-factors which enhance APC anticoagulant activity in-vivo, preferably wherein the lipid co-factor is Protein S.

5. Protein C variant according to any of the preceding claims wherein the variant has reduced sensitivity to lipids.

6. Protein C variant according to claim 5 wherein the lipid is phosphatidylethanolamine (PE) and/or glucosylceramide (GlyCer).

7. Protein C variant according to claim 6 wherein the interaction of APC with PE and GlyCer is reduced or eliminated.

8. Protein C variant according to any of claims 4 to 7 wherein the interaction of APC with Protein S is reduced or eliminated

9. Protein C variant according to any of the preceding claims which has one or more amino acid residue substitutions in the APC γ-carboxyglutamic acid (Gla) domain.

10. Protein C variant according to claim 9 comprising the substitution I18V.

11. Protein C variant according to claim 5 wherein the lipid is cardiolipin and/or high density lipoprotein (HDL).

12. Protein C variant according to any of the preceding claims in an activated form.

13. A pharmaceutical composition comprising protein C variant or activated protein C variant according to any of claims 1 to 12 with a suitable pharmaceutical excipient.

14. Protein C variant according to any of the preceding claims for use in therapy.

15. Use of protein C variant according to any of claims 1 to 12 for the manufacture of a medicament for the treatment of anti-inflammatory conditions, such as severe sepsis, inflammatory bowel disease or ischemic stroke.
